# EUROPEAN PATENT SPECIFICATION

(11) **EP 2 419 407 B1**
(45) Date of publication and mention of the grant of the patent: **14.11.2012**
(21) Application number: 09776534.1
(22) Date of filing: 15.04.2009
(51) Int. Cl.: C07D 209/24

(54) **IMPROVED PROCESS FOR THE PREPARATION OF FLUVASTATIN AND SALTS THEREOF**
VERBESSERTES VERFAHREN ZUR HERSTELLUNG VON FLUVASTATIN UND SALZEN DAVON
PROCÉDÉ AMÉLIORÉ DE PRÉPARATION DE FLUVASTATINE ET DE SELS DE CELLE-CI

(43) Date of publication of application: 22.02.2012
(73) Proprietor: Pharmathen S.A., 15351 Pallini Attikis (GR)
(72) Inventor: KOFTIS, Theoharis, V., GR-57001 Thessaloniki (GR); PANAGIOTIDIS, Theodoros, GR-546 41 Thessaloniki (GR); SONI, Rohit, Ravikant, GR-57001 Thessaloniki (GR); LITHADIOTI, Alexandra, GR-570 01 Thessaloniki (GR)
(86) International application number: PCT/EP2009/002745
(87) International publication number: WO 2010/118757

(56) References cited:
- EP-A- 1 847 529
- WO-A-2004/113291

## Description

### TECHNICAL FIELD OF THE INVENTION

The present invention relates to an improved process for the preparation of Fluvastatin and pharmaceutical acceptable salts or derivatives thereof and in particular to a one-pot process for large scale production of Fluvastatin or salts thereof and pharmaceutical preparations containing said compounds.

### BACKGROUND OF THE INVENTION

Fluvastatin belongs to a class of drugs called statins, which act as inhibitors of 3-hydroxy-3-methylglutaryl-coenzyme-A (HMG-CoA) reductase. Statins are effective in reducing low-density lipoprotein (LDL) particles concentration in the blood stream and used in the treatment of hypercholesterolemia and hyperlipoproteinemia. Moreover, they are very useful in preventing coronary heart disease (CHD), which continues to be a major health problem in developed countries.

Fluvastatin is used in the form of Fluvastatin sodium, which is more desirable since it can be more efficiently formulated. This is important, because formulations need to meet certain pharmaceutical requirements and specifications. Fluvastatin sodium can be easily formulated in the form of tablets, capsules, lozenges, powders, and other forms for oral administration.

Fluvastatin sodium is chemically designated as sodium (3*RS*, 5SR, 6E)-7-[3-(4-fluorophenyl)-1-(1-methylethyl)-1*H*-indol-2-yl]-3, 5-dihydroxyhept-6-enoate.

Fluvastatin is a racemic mixture of the (3R, 5S) and (3S, 5R) enantiomers and present the following structural formula:

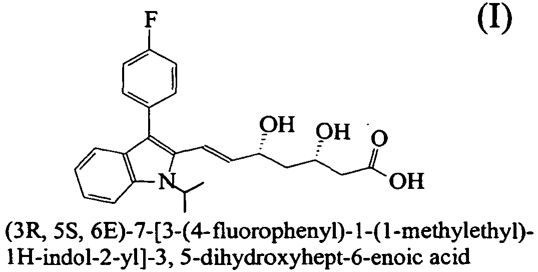

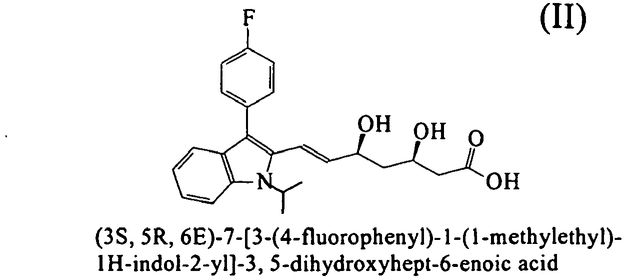

These two isomers are also called *syn*-diol isomers and the other isomers of the same chemical structure are called *anti*-isomers.

Fluvastatin and its sodium salt and methods for the preparation thereof, were first disclosed in EP-B-114 027, wherein Fluvastatin sodium is obtained by hydrolysis of fluvastatin methyl ester with sodium hydroxide in ethanol followed by lyophilization. Further, several processes for the preparation of Fluvastatin sodium have also been disclosed, including a number of polymorphic forms thereof. However, lyophilization is an expensive and time-consuming process on large scale.

Prior art processes for the preparation of Fluvastatin sodium present the disadvantage of non-satisfactory yield of the product. Furthermore, the compound often comprises significant amounts of impurities and the chemical reactions may require a long period of time to be completed.

It is also known that pure Fluvastatin sodium exists in amorphous form and that fluvastatin sodium hydrates/solvates can easily convert into other crystalline forms during storage. Further, purity is of highly importance with regard to active pharmaceutical ingredients such as fluvastatin, which must be considered on long term basis since impurity may accumulate with time and cause undesired side effects. Therefore, for the sake of stability and chemical purity, it is advantageous to manufacture anhydrous fluvastatin sodium in substantially pure amorphous form.

WO 2006/038219 discloses a process for the preparation of amorphous fluvastatin sodium, in which methanol solutions of NaOH and *tert*-butyl ester of Fluvastatin are combined and stirred. When the reaction is complete, the reaction mass is twice concentrated, dissolved in methanol and filtered. The yield of said process is very low, less than 50%. Moreover, NaOH is added in excess, but aqueous work up for removing inorganic impurities is not carried out.

WO 2004/113292 discloses a process for the preparation amorphous fluvastatin sodium comprising dissolving Fluvastatin methyl ester in acetone, adding a solution of NaOH in methanol, stirring the reaction mixture at room temperature overnight and isolating the product by filtration under nitrogen. The final product is dried for 24 hours. According to another embodiment, fluvastatin sodium is dissolved in 1,4 dioxane at elevated temperature and then the solution is cooled to room temperature and stirred for 70 hours to induce precipitation of amorphous fluvastatin sodium. Yield in both processes is 90% and both processes require long reaction time and no additional work up for removing impurities is carried out. In addition, the use of 1, 4 dioxane in large scale and at elevated temperature is extremely hazardous, as this solvent is listed as probable carcinogenic by the International Agency for Research on Cancer. These processes are obviously not feasible for industrial practice.

EP-A-1847529 discloses a selective hydrolysis process for large scale production of fluvastatin, wherein only the desired *syn*-isomer is hydrolyzed and the unwanted anti-isomers remain unchanged and can be easily removed by extraction. The diastereomeric purity of the resulting compound is controlled by two factors, namely the diastereomeric purity of the reacting ester and the quantities of the reagents and solvents. Although said process seems to be less expensive for achieving stereo-selection in large scale, however no isolation of product through filtration is described, rather than distillation of solvents till damp solid is obtained, leaving the content of other impurities in question.

Although each of the above patents represents an attempt to overcome the use of costly and hazardous material, there still exists a need for a cost-effective and safer process for large scale production which provides higher yield with higher purity.

### SUMMARY OF THE INVENTION

It is, therefore, an object of the present invention to provide an improved process for the preparation of fluvastatin or pharmaceutical acceptable salts thereof or its derivatives, which overcomes the deficiencies of the prior art processes and results to a cost effective industrial production without scarifying the yield and quality of the product.

Another object of the present invention is to provide an improved method for the preparation of Fluvastatin or salts thereof or its derivatives by selecting the appropriate reactants, catalysts, solvent systems and conditions used during the organic reactions, so that the purity (both chemical purity and optical purity) and yield of the reaction are increased and the presence of any contaminants and formed by-products is minimized.

Further object of the present invention is to provide an improved method for the preparation of Fluvastatin or salts thereof, or its derivatives, by using milder and safer reaction conditions that helps protecting the environment and the personnel.

In accordance with the above objects of the present invention, a process for the preparation of Fluvastatin or pharmaceutically acceptable salts thereof or its derivatives is provided comprising the following steps:
a) dissolution of 1, 1-dimethylethyl (3*R**,5*S**,6*E*)-7-[3-(4-fluorophenyl)-1-(1-methylethyl)-1*H-*indol-2-yl]-3,5-dihydroxyhept-6-enoate, also known as *tert*-butyl ester of fluvastatin, in a mixture of toluene and methanol;
b) addition of aqueous solution of NaOH into the obtained solution from step a);
c) phase separation of the reaction mass;
d) concentration of the combined aqueous layers to obtain an aqueous suspension;
e) filtration of the suspended precipitated solid and wash with water to obtain a thick paste;
f) dissolution of the solid in THF, filtration through celite or cartridge filter and addition of cyclohexane to the clear THF solution and stir the precipitated product for approximately three hours and cool down gradually to about 25° - 30°C;
g) filtration and wash of the obtained solid with cyclohexane; and
h) dissolution the obtained wet cake in methanol, filtration and spray drying.

Preferred embodiments of the present invention are set out in dependent claims 2 to 14.

Other objects and advantages of the present invention will become apparent to those skilled in the art in view of the following detailed description.

### DETAILED DESCRIPTION OF THE INVENTION

The present invention relates to an improved process for the preparation of Fluvastatin and pharmaceutically acceptable salts thereof in a stable form, which is characterized in substantially shorter reaction time, milder and safer reaction conditions, without scarifying the yield and quality of the product and low cost of reactants and reagents.

According to the present invention, the process for the preparation of Fluvastatin and pharmaceutically acceptable salts thereof, or its derivatives comprises the following steps:

A compound of formula (III), 1,1-dimethylethyl (3*R**,5*S**,6*E*)-7-[3-(4-fluorophenyl)-1-(1-methylethyl)-1*H-*indol-2-yl]-3,5-dihydroxy-hept-6-enoate, *i.e. tert*-butyl ester of fluvastatin is added into a mixture of toluene and methanol (about 2:1, v/v) and the mixture is stirred at about 25°C - 30°C until a clear solution is obtained;

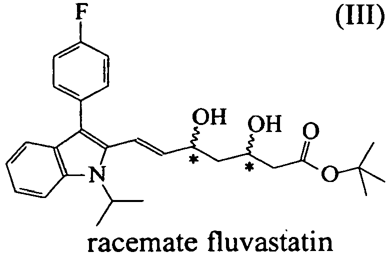

An aqueous solution of NaOH (10%, w/v) is added to the solution obtained in step a) while stirring and keeping the solution temperature at about 25°C-30°C for approximately 3-5 hours. The reaction mass is allowed to settle into two layers and subsequently the aqueous layer is washed by using toluene and the combined organic layer is extracted with DM water and thereafter the organic layers are discarded.

Combined aqueous layers are being concentrated under vacuum to about 3.0-3.5 times of the volume of the starting material and the aqueous suspension of the precipitated solid is stirred for approximately three hours and gradually cooled to about 15-20°C.

The obtained precipitation is filtered and washed with water in order to obtain a thick paste, and the thick paste is dissolved in THF at about 50°C and filtered through celite or cartridge filter. To the obtained clear filtrate, cyclohexane is added and the precipitated product is stirred for approximately three hours and gradually cooled to about 25-30°C.

The solids are filtered and washed using cyclohexane and the wet cake is dissolved in methanol, and thereafter filtered through a 5 micron filter cartridge and spray dried at about 100°C to obtain anhydrous fluvastatin sodium amorphous in more than 99.9% purity and less than 0.5% anti-isomers.

The *t*-butyl ester of Fluvastatin is selected as the key starting material for the present process and prepared according to prior art processes, with minor modifications in order to provide ester in high quality and yield in respect of the anti-diastereomer [(3*RS*,5*RS*,6*E*)-7-[3-(4-fluorophenyl)-1-(1-methylethyl)-1*H*-indol-2-yl]-3,5-dihydroxyhept-6-enoic acid 1,1-dimethylethyl ester]. Further, (5RS, E)-t-butyl 7-(3-(4-fluorophenyl)-1-methylethyl-1H-indol-2-yl)-5-hydroxy-3-oxo-6-heptanoic acid 1,1-dimethylethyl ester is obtained by an aldol-type condensation of E-3-(3-(4-Fluorophenyl)-1-(1-methylethyl)-1H-indol-2-yl)prop-3-enaldehyde with dimethyl ethyl acetoacetate according to the method described in example 1e of EP 114 027.

The diastereoselective reduction of (5RS,E)-t-butyl 7-(3-(4-fluorophenyl)-1-methylethyl-1H-indol-2-yl)-5-hydroxy-3-oxo-6-heptanoic acid 1,1-dimethylethyl ester is carried out using dialkylalkoxy borane and sodium borohydride, which gives a high syn-diol to anti diastereomeric ratio for the obtained diol.

The hydrolysis of the ester as mentioned in the process steps (a) and (b) is a biphasic system which comprises the use of methanol/water/ toluene, said system is quite efficient for the hydrolysis, due to the high solubility of both starting material and product.

Process step (c) assures the removal of any organic soluble impurities from the final product, wherein process steps (d) and (e) eliminate the inorganic water soluble impurities. In case any organic impurities passed through step (e), said potential impurities are removed in step (f), wherein an organic solvent is used to dissolve fluvastatin sodium and a filtration of the solution is carried out.

During process steps (g) and (h) the purity of fluvastatin sodium in respect to organic impurities - including the anti-diastereomer - is improved and subsequently the dissolution in methanol and spray drying of the material provides amorphous fluvastatin sodium with a purity greater than 99.8% and in high yield (greater than 90%).

This robust process is taking care of all inorganic and organic purities, providing highly pure material in excellent yield and for multi-tons industrial production. The only impurity that is eventually detectable is the anti-diastereomer of fluvastatin sodium, which derives from the hydrolysis of the anti-diastereomer of the *t*-butyl ester.

The diastereomeric purity of the *t*-butyl ester is enhanced with additional re-crystallizations. It has been observed that after two successive re-crystallizations about less than 0.7 - 0.8% of the anti-diastereomer is present, and after three successive re-crystallizations the *t*-butyl ester obtained has less than 0.1-0.2 % of its anti diastereomer.

Moreover, the obtained final product has less than 0.4-0.5 % and 0.1-0.2 % of the anti-diastereomer, respectively.

According to the present invention the following analytical method has been developed for the detection and quantification of the anti-diastereomer of the fluvastatin *t*-butyl ester. Said method comprises:
a. injecting a sample of fluvastatin *t*-butyl ester in methanol on a Symmetry Shield RP C₁₈ 5 µm column (4.6x 100 mm), and
b. gradient eluting the sample with a mixture of acetonitrile/THF/phosphate buffer (pH 8).

According to the method of the present invention, the content of the anti-diastereomer at the *t-*butyl ester stage can be easily controlled, and hence the quality of the final Fluvastatin sodium is controlled.

In addition, prior to the hydrolysis of the *t*-butyl ester, purification can be carried out in order to enhance the quality of the final Fluvastatin sodium. The cost in yield of the extra purification is not significant since there is a 95% w/w recovery of material.

The process of the present invention will be demonstrated in more details with reference to the following examples, which are provided by way of illustration only and should not be construed as limit to the scope of the reaction in any manner.

### Example 1: Preparation of 1, 1-dimethylethyl (3R, 5S, 6E)-7-[3-(4-fluorophenyl)-1-(1-methylethyl)-1H-indol-2-yl]-3, 5-dihydroxyhept-6-enoate

26 g of NaH is dissolved in 200 ml THF at 20-30°C and stirred for 10-15 min. This solution is cooled to about 0°C and a solution of 106 ml of *tert*-butyl acetoacetate in 200 ml THF is added over a period of approximately 15 min while stirring and maintaining the temperature below 5°C. The mixture is stirred below 5°C for an additional 2 hours followed by adding 278 ml of *n*-BuLi (1.6 M in hexane) over a period of 30-60 min and stirred below 5°C for another 120 min. The reaction mass is cooled to about -10°C, a solution of 100 g 3-(4-fluorophenyl)-1-(methylethyl)-1*H*-indole-2-carboxyaldehyde (compound 1, formula III) in 230 ml THF is added over a period of 15-30 min, maintaining the temperature below -5°C. The reaction mass is stirred at below -5°C for 60 min, quenched with app. 200 - 250 ml 6.0 N HCl, while keeping the temperature below 10°C (keeping pH between 6.0 - 7.0) and then 50 ml H₂O is added. This reaction mass is stirred for 15-30 min.

The reaction mass is phase-separated into two layers. The aqueous layer is extracted twice with toluene (250 ml and 150 ml). The combined organic layers are backed washed with brine (200 ml) and concentrated under reduced pressure affording compound 2 (formula IV) as thick oil. Dissolving compound 2 in 800 ml THF and 200 ml methanol, adding 76 ml of diethylmethoxyborane (50 % in THF) and the reaction mass is cooled to -78°C. 20 g of sodium borohydride is added and, after completion of reaction, the mass is warmed up to -25 °C. Quenched with 800 ml water and 100 ml of hydrogen peroxide (30% w/v) and the reaction mass is warmed to 25°C. 500 ml toluene is added. The reaction mass is filtered to remove the inorganic solids. The filtrate is transferred into a separatory funnel for phase separation.

The inorganic solids are suspended in 300 ml toluene, heated to 35-40°C and filtered off. The mother liquid is used to extract the aqueous phase obtained from the phase separation. The organic layers are combined and washed with 200 ml aq. sodium thiosulfate (2.5% w/v) and concentrated to give a crude solid mass that is re-crystallized twice with mixture of toluene and cyclohexane (150ml/600ml the first, 200ml/500ml the second) to afford 105-110g of (3*R,5S,6E*)-7-[3-(4-fluorophenyl)-1-(1-methylethyl)-1*H*-indol-2-yl]-3,5-dihydroxyhept-6-enoate in 99.8 - 99.9 % purity and with the anti-isomer less than 0.7 %.

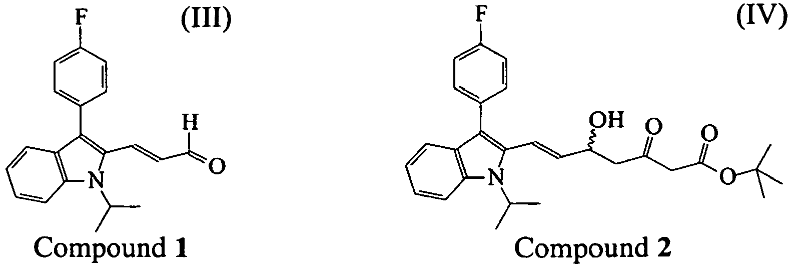

### Example 2: Preparation of Fluvastatin sodium amorphous

To a three-necked round-bottom flask, 100g of (3*R*,5*S*,6*E*)-7-[3-(4-fluorophenyl)-1-(1-methylethyl)-1*H*-indol-2-yl]-3,5-dihydroxyhept-6-enoate, prepared according to the Example 1 of the present invention, is added into a mixture of 1100ml toluene and 500ml methanol under stirring at 25°C-30°C. The mixture is stirred at the same temperature until the material dissolves (takes about 15min). Then, 100ml of 10% NaOH (w/v) solution is added within 1-2 minutes at 25-35°C.

After stirring at 25-30°C for about 5 hours, the reaction mass is transferred to a separatory funnel. The separated aqueous layer is washed with 200ml toluene. The organic layers are combined and extracted with 400ml DM Water. The organic layer is discarded. The combined aqueous layers are transferred again into a separatory funnel to remove any residual organic solvent and the aqueous layer is concentrated under vacuum to about 2.8-3.2 volumes (referring to the starting material). The suspension is stirred for three hours while it is gradually cooled to 15°C-20°C. The precipitated solid is filtered, washed with 50ml H₂O and dissolved in 200ml of THF at 50°C. 1500ml of cyclohexane is added to the clear resulting solution. The suspension is stirred for three hours while is gradually cooled to 25°C-30°C. The precipitated solid is filtered and washed with 3x100ml cyclohexane. The resulting wet cake is dissolved in 1000 ml methanol and spray-dried at 100°C to provide 85-90 gr Fluvastatin sodium amorphous having purity higher than 99.9 % and with the anti-isomers less than 0.5 %.

### Example 3: Preparation of 1, 1-dimethylethyl (3R, 5S, 6E)-7-[3-(4-fluorophenyl)-1-(1-methylethyl)-1H-indol-2-yl]-3, 5-dihydroxyhept-6-enoate

The product prepared according to the process of Example 1 is re-crystallized once more using with the same solvent system as in the previous crystallization of Example 1 of the present invention.

The yield obtained is 95 g of pure *(3R, 5S, 6E)-7-[3-(4-fluorophenyl)-1-(1-methylethyl)-1H-indol-2-yl]-3, 5-dihydroxyhept-6-enoate* having purity higher than 99.9 % and the anti-isomers less than 0.2 %.

### Example 4: Preparation of Fluvastatin sodium amorphous

The process according to Example 2 is followed by using the product prepared according to the process of Example 3 of the present invention.

The yield obtained is 85 - 90 gr Fluvastatin sodium amorphous in more than 99.9% purity and having less than 0.1 % of the anti-isomers.

The present invention describes a large scale manufacture process for the preparation of fluvastatin sodium in a stable form and in high purity at relative low production cost compared to the available processes for producing similar products.

Therefore, the amorphous fluvastatin sodium according to the process of the present invention is obtained in excellent yield (above 90%), in high purity (above 99.8%) directly from *tert*-butyl ester of fluvastatin, without any isolation and drying at any intermediate stage. This is an advanced industrial process capable of providing high quality fluvastatin sodium in multi tones scale, meeting by far the specifications of US Pharmacopeia.

While the present invention has been described with respect to the particular embodiments, it will be apparent to those skilled in the art that various changes and modifications may be made in the invention without departing from the spirit and scope thereof, as defined in the appended claims.

## Claims

1. A process for the preparation of amorphous fluvastatin sodium, which comprises:
a) dissolution of 1, 1-dimethylethyl (3*R**,5*S**,6*E*)-7-[3-(4-fluorophenyl)-1-(1-methylethyl)-1*H-*indol-2-yl]-3,5-dihydroxyhept-6-enoate, also known as *tert*-butyl ester of fluvastatin, in a mixture of toluene and methanol;
b) addition of aqueous solution of NaOH into the obtained solution from step a);
c) phase separation of the reaction mass;
d) concentration of the combined aqueous layers to obtain an aqueous suspension;
e) filtration of the suspended precipitated solid and wash with water to obtain a thick paste;
f) dissolution of the solid in THF, filtration through celite or cartridge filter and addition of cyclohexane to the clear THF solution and stir the precipitated product for approximately three hours and cool down gradually to about 25° - 30°C;
g) filtration and wash of the thus obtained solid with cyclohexane; and
h) dissolution the obtained wet cake in methanol, filtration and spray drying.

2. The process according to claim 1, wherein the concentration of the solution of step a) is in the range from 4% to 10%, preferably 6.5%.

3. The process according to claim 1, wherein the ratio of toluene to methanol is in the range from 5:1 to 1:5 (v/v), preferably 2:1 (v/v).

4. The process according to claim 1, wherein the molar ration of *tert*-butyl fluvastatin to NaOH is slightly lower than 1.

5. The process according to claim 1, wherein the reaction mass of step b) is being maintained at about 25 - 30°C for approximately 3 to 5 hours.

6. The process according to claim 1, wherein during phase separation, the aqueous layer is washed with toluene and the organic layer is extracted with DM water, said aqueous layers are combined and said organic layers are discarded.

7. The process according to claim 1, wherein the combined aqueous layers are concentrated under vacuum to about 1/4 of its volume and 2.8 - 3.2 times of the volume of the starting material.

8. The process according to claim 1, wherein the aqueous suspension obtained in step d) is stirred for approximately 3 hours and gradually cooled down to about 15° - 20°C.

9. The process according to claim 1, wherein the ratio of THF and cyclohexane is in the range from 1:5 to 1: 10 (v/v), preferably 1:7.5 (v/v).

10. The process according to claim 1, wherein the spray-drying is conducted at 100°C.

## Patentansprüche

1. Ein Verfahren zur Herstellung von amorphen Fluvastatin-Natrium, umfasst:
a) 1, 1-Dimethylethyl (3R *, 5S *, 6E) -7 - [3 - (4-Fluorphenyl) -1 -(1-methylethyl)-1H-indol-2-yl]-3,5 -dihydroxyhept-6-enoat, auch als tert-Butyl-Ester von Fluvastatin bekannt ist, in einer Mischung von Toluol und Methanol auflösen;
b) einer wäßrigen Lösung von NaOH in die erhaltene aus Schritt a) Lösung zugaben;
c) der Phase der Reaktionsmasse abtrennen;
d) Konzentration der vereinigten wässrigen Schichten eines wässrigen Suspension zum erhalten;
e) der suspendierten ausgefallene Feststoff filtrieren und mit Wasser waschen um einer dicken Paste zu erhalten;
f) der Feststoff in THF auflösen, durch Celite oder Patronenfilter filtrieren und Cyclohexan zu der klaren THF Lösung zugeben und des ausgefallenen Produkts für etwa drei Stunden rühren und allmählich auf etwa 250 - 30 ° C abkühlen;
g) filtrieren und des erhaltenen Feststoffs mit Cyclohexan waschen und
h) des erhaltenen feuchten Masse in Methanol lösen, filtrieren und sprühtrocknen.

2. Das Verfahren nach Anspruch 1, wobei die Konzentration der Lösung von Schritt a) im Bereich von 4% bis 10%, vorzugsweise 6,5% liegt.

3. Das Verfahren nach Anspruch 1, wobei das Verhältnis von Toluol zu Methanol im Bereich von 5:1 bis 1:5 (v / v), bevorzugt 2:1 (v / v) ist.

4. Das Verfahren nach Anspruch 1, wobei das molare Verhältnis von tert.-Butyl Fluvastatin zu NaOH etwas geringer als 1 ist.

5. Das Verfahren nach Anspruch 1, wobei die Reaktionsmasse von Schritt b) für etwa 3 bis 5 Stunden bei etwa 25 - 30 ° C gehalten wird.

6. Das Verfahren nach Anspruch 1, wobei während der Phasentrennung die wässrige Schicht mit Toluol gewaschen wird und die organische Schicht mit DM Wasser extrahiert wird, wobei die wässrige Schichten kombinieren werden und die organischen Schichten verworfen werden.

7. Das Verfahren nach Anspruch 1, wobei die vereinigten wässrigen Schichten im Vakuum auf etwa 1/4 seines Volumens und 2,8 - 3,2-fache des Volumens des Ausgangsmaterials konzertieren werden.

8. Verfahren nach Anspruch 1, wobei die wässrige Suspension von Schritt d) für ca. 3 Stunden gerührt und allmählich auf etwa 15° - 20° C abgekühlt wird.

9. Das Verfahren nach Anspruch 1, wobei das Verhältnis von THF und Cyclohexan im Bereich von 1:5 bis 1: 10 (v / v), vorzugsweise 1:7,5 (v / v) ist.

10. Verfahren nach Anspruch 1, wobei die Sprühtrocknung bei 100 ° C durchgeführt wird.

## Revendications

1. Un procédé pour la préparation de la fluvastatine de sodium amorphe, qui comprend:
a) la dissolution du 1,1-diméthyléthyl (3R*,5S*,6E)-7-[3-(4-fluorophényl)-1-(1-méthyléthyl)-1H-indol-2-yl]-3,5-dihydroxyhept-6-énoate, également connu comme l'ester *tert*-butylique de la fluvastatine, dans un mélange de toluène et de méthanol;
b) l'addition d'une solution aqueuse de NaOH à la solution obtenue à l'étape a);
c) la séparation des phases de la masse réactionnelle;
d) la concentration des phases aqueuses combinées pour obtenir une suspension aqueuse;
e) la filtration de la suspension de solide précipité et le lavage avec de l'eau pour obtenir une pâte épaisse;
f) la dissolution du solide dans du THF, la filtration sur célite ou cartouche de filtre et l'addition de cyclohexane à la solution claire de THF, et agiter le produit précipité pendant environ trois heures et refroidir graduellement jusqu'à environ 25°-30°C;
g) la filtration et lavage du solide ainsi obtenu avec du cyclohexane; et
h) la dissolution du gâteau humide obtenu dans le méthanol, filtration et séchage par pulvérisation.

2. Le procédé selon la revendication 1, dans lequel la concentration de la solution de l'étape a) est de l'ordre de 4% à 10%, préférablement 6.5%.

3. Le procédé selon la revendication 1, dans lequel le rapport entre le toluène et le méthanol est de l'ordre de 5:1 à 1:5 (v/v), préférablement 2:1 (v/v).

4. Le procédé selon la revendication 1, dans lequel le rapport molaire de *tert*-butyle de fluvastatine au NaOH est légèrement inférieure à 1.

5. Le procédé selon la revendication 1, dans lequel la masse réactionnelle de l'étape b) est maintenue à environ 25-30°C pour approximativement 3 à 5 heures.

6. Le procédé selon la revendication 1, dans lequel pendant la phase de séparation, la phase aqueuse est lavée avec du toluène et la phase organique est extraite avec de l'eau DM, les dites phases aqueuses sont réunies et les dites phases organiques sont éliminées.

7. Le procédé selon la revendication 1, dans lequel les phases aqueuses combinées sont concentrées sous vide à environ ¼ de leur volume et de 2.8 à 3.2 fois le volume de la matière de départ.

8. Le procédé selon la revendication 1, dans lequel la suspension aqueuse obtenue à l'étape d) est agitée pendant approximativement 3 heures et graduellement refroidie jusqu'à 15°-20°C.

9. Le procédé selon la revendication 1, dans lequel le rapport entre THF et cyclohexane est de l'ordre de 1:5 à 1:10 (v/v), préférablement 1:7.5 (v/v).

10. Le procédé selon la revendication 1, dans lequel le séchage par pulvérisation est effectué à 100°C.
